# EUROPEAN PATENT SPECIFICATION

(11) **EP 2 009 444 B1**
(45) Date of publication and mention of the grant of the patent: **29.02.2012**
(21) Application number: 07741215.3
(22) Date of filing: 06.04.2007
(51) Int. Cl.: C12Q 1/26

(54) **NON-INVASIVE TEST METHOD FOR NON-ALCOHOLIC STEATOHEPATITIS BASED ON CYTOCHROME-C QUANTIFICATION**
VERFAHREN ZUR NICHTINVASIVEN UNTERSUCHUNG NICHT-ALKOHOLISCHER STEATOHEPATITIS MITTELS QUANTIFIZIERUNG VON CYTOCHROM-C
METHODE D'EXAMEN NON INVASIVE DE LA STEATO-HEPATITE NON ALCOOLIQUE PAR QUANTIFICATION DE LA CYTOCHROME C

(30) Priority: 06.04.2006 JP 2006105516
(43) Date of publication of application: 31.12.2008
(73) Proprietor: Eisai R&D Management Co., Ltd., Tokyo 112-8088 (JP); National University Corporation Kumamoto University, Kumamoto-shi Kumamoto 860-8555 (JP)
(72) Inventor: SASAKI, Yutaka, Kumamoto-shi, Kumamoto 860-0811 (JP); ASHIHARA, Hiroshi, Kumamoto-shi, Kumamoto 860-0811 (JP); NAGAHAMA, Hiroyasu, Kumamoto-shi, Kumamoto 860-0811 (JP); AOYAMA, Muneo, Tsukuba-shi, Ibaraki 300-2635 (JP)
(74) Representative: Dean, John Paul
(86) International application number: PCT/JP2007/057779
(87) International publication number: WO 2007/116975

(56) References cited:
- EP-A- 1 229 328
- EP-A- 1 873 523
- WO-A-2007/145274
- ISAO SAKAIDA ET AL: "Cytochrome c is a possible new marker for fulminant hepatitis in humans" JOURNAL OF GASTROENTEROLOGY, SPRINGER-VERLAG, TO, vol. 40, no. 2, 1 February 2005 (2005-02-01), pages 179-185, XP019373148 ISSN: 1435-5922
- SANTAMARIA E ET AL: "Functional proteomics of nonalcoholic steatohepatitis and hepatocarcinoma. Mechanisms and biomarkers" JOURNAL OF HEPATOLOGY, vol. 40, no. Suppl. 1, April 2004 (2004-04), page 175, XP002536633 & ABSTRACTS OF THE 39TH ANNUAL MEETING OF THE EUROPEAN ASSOCIATION FOR THE STUDY OF THE LIVER; BERLIN, GERMANY; APRIL 14-18, 2004 ISSN: 0168-8278
- PESSAYRE D ET AL: "NASH: a mitochondrial disease" JOURNAL OF HEPATOLOGY, MUNKSGAARD INTERNATIONAL PUBLISHERS, COPENHAGEN, DK, vol. 42, no. 6, 1 June 2005 (2005-06-01), pages 928-940, XP025293805 ISSN: 0168-8278 [retrieved on 2005-06-01]
- KAWANAKA M. ET AL. SHINDAN TO CHIRYO vol. 93, no. 12, 2005, pages 2165 - 2168, XP003018075
- OSUGA M. ET AL. SHINDAN TO CHIRYO vol. 93, no. 12, 2005, pages 2171 - 2177, XP003018076

## Description

### Technical Field

The present invention is in the field of a method for non-invasively testing non-alcoholic steatohepatitis, and use of a test kit for the method.

### Background Art

Chronic liver diseases shift to chronic hepatitis, liver cirrhosis, and finally hepatoma. The major cause thereof is hepatitis C virus or hepatitis B virus infection. In addition, non-alcoholic steatohepatitis (NASH), showing pathological conditions characterized by alcoholic hepatitis-like tissue images, behind which fatty liver lies, without evident drinking history, comes to attract attentions in recent years, and it has also been elucidated that non-alcoholic steatohepatitis progresses to liver cirrhosis and hepatoma.

For the pathology of non-alcoholic steatohepatitis, the two hit theory is estimated, that is, following fatty change of the liver as the first step (first hit), hepatocellular damage (apoptosis) and fibrosis are induced by addition of various stresses as the second step (second hit). Strong correlation of non-alcoholic fatty liver diseases (NAFLDs) including non-alcoholic steatohepatitis with insulin resistance syndromes such as obesity, diabetes and hyperlipidemia is evident, and they are the commonest hepatic diseases in the United States with being supposed that 70% of pycnic types are complicated by fatty liver. Furthermore, since it has been reported that 10% of advanced pycnic types (BMI>30) suffer from non-alcoholic steatohepatitis, it is estimated that 250,000 or more of non-alcoholic steatohepatitis patients exist also in Japan.

At present, hepatitis C virus infection accounts for a little less than about 80% of the causes of onset of hepatoma, and non-alcoholic steatohepatitis accounts for only several percents of the same (Non-patent document 1). However, in contrast to the estimation that onset risk of hepatoma due to hepatitis C virus infection would be decreased in future by screening of blood for transfusion for hepatitis C virus, and development of diagnostic agents for hepatitis C and therapeutic agents for the same such as interferons, hepatoma due to non-alcoholic steatohepatitis is expected to increase also in Japan with increase of insulin resistance syndrome patients due to westernization of the dietary habits.

However, since any specific marker does not exist for non-alcoholic steatohepatitis, it is currently inevitable for diagnosis thereof to use pathological diagnosis based on invasive liver biopsy.
Non-patent document 1: Bugianesi E et al., Gastroenterology, 2002, 123:134-140
Santamaria et al., 2004 discloses a method to provide biomarkers in liver samples, such as cytochrome C oxidase I and II (COX I and COX II), to detect Non-Alcoholic Steatohepatitis (NASH).

### Disclosure of the Invention

An object of the present invention is to develop a method for non-invasive test of non-alcoholic steatohepatitis.

It is considered that hepatocellular damages (apoptosis) induced by various stresses added to fatty liver is involved in non-alcoholic steatohepatitis (Ribeiro PS et al., Am. J. Gastroenterol., 2004 Sep., 99(9):1708-17).

The inventors of the present invention considered that the stress-dependent hepatocellular damages (apoptosis) arising in non-alcoholic fatty hepatitis could be evaluated on the basis of blood cytochrome c level, as the measurement of apoptosis arising in the living bodies of liver haemophagocytic syndrome (HPS), GVHD, acute lymphatic leukemia and influenzal encephalitis patients disclosed in WO01/35093.

Then, they found that blood cytochrome c levels quantified for non-alcoholic steatohepatitis patients were higher than those for healthy persons, and that the quantified blood cytochrome c values correlated with fat deposition rates in hepatocytes, and accomplished the present invention.

The present invention is described in claims 1-6.

### Brief Description of the Drawings

[Fig. 1] Fig. 1 shows quantification results for cytochrome c amounts in blood serum of non-alcoholic steatohepatitis patients and healthy persons.
[Fig. 2] Fig. 2 shows relationship between fat deposition rates in hepatocytes and quantified values of cytochrome c in blood serum.

### Best Mode for Carrying out the Invention

Hereafter, embodiments of the present invention will be explained in detail.
In this specification, blood means blood, plasma or serum collected from a living body.

As the method for measuring cytochrome c, immunochemical method, electrophoresis, chromatography, and so forth can be contemplated, and an immunochemical method is preferred in view of sensitivity and simplicity.

The immunochemical method referred to herein is a method for quantifying cytochrome c by using an antibody against cytochrome c. The immunochemical method include various methods such as the competitive method in which cytochrome c is labeled, the sandwich method in which the antibody is labeled and the latex bead method in which aggregation of antibody-coated beads is observed, and any immunochemical method may be used for the present invention so long as a method using an antibody against cytochrome c is chosen. The antibody may be a monoclonal antibody or a polyclonal antibody. The labeling method also includes various methods such as labeling with a radioactive isotope, labeling with an electrochemiluminescent compound, labeling with fluorescence, labeling with an enzyme and labeling with biotin, and the present invention is not limited to any one of these examples. The methods for preparing and labeling antibodies are described in, for example, Lecture of Biochemical Experiment Second Series, vol. 5, Methods for Immunobiochemical Research (edited by Japanese Biochemical Society, published by Tokyo Kagaku Dojin), and Lecture of Biochemical Experiment New Edition, vol. 12, Molecular Immunology III (edited by Japanese Biochemical Society, published by Tokyo Kagaku Dojin).

As an example of the immunochemical method for quantifying cytochrome c, the sandwich method will be explained below in the order of the steps thereof.

1) Antibodies against cytochrome c are immobilized on beads or cup. The antibodies to be immobilized may be either polyclonal antibodies or monoclonal antibodies, so long as antibodies providing required sensitivity, preferably sensitivity enabling measurement of 0.5 ng/mL, more preferably 0.1 ng/mL, of cytochrome c are chosen. As the monoclonal antibodies, commercially available antibodies such as Clone: 6H2.B4 (Becton Dickinson) and Clone: 2B5F8 (TECHNE) may be used. The beads may be micro beads, and in such a case, micro beads consisting of magnetic substance are preferred. The immobilization may be attained by either covalent bond or non-covalent bond. In order to block nonspecific bonding sites on the beads or cup, a blocking treatment is usually performed with a protein such as bovine serum albumin (BSA) and casein or a surfactant such as Tween 20.

2) A sample is added to the beads or cup after the sample is diluted with a buffer solution containing a protein such as BSA and casein or a surfactant such as Tween 20, if necessary. Cytochrome c of a known amount is similarly diluted and added.

3) After washing the beads or cap with a buffer solution containing a surfactant such as Tween 20, if possible, labeled anti-cytochrome c antibodies, diluted with a buffer solution containing a protein such as BSA and casein or a surfactant such as Tween 20, if possible, are added to the beads or cup. As the labeled antibodies, either polyclonal antibodies or monoclonal antibodies may be acceptable, so long as antibodies providing required sensitivity, preferably sensitivity enabling measurement of 0.5 ng/mL, more preferably 0.1 ng/mL, of cytochrome c are used. When the antibodies to be immobilized are monoclonal antibodies, monoclonal antibodies of a clone different from that of the monoclonal antibodies to be immobilized are preferably used for the labeled monoclonal antibodies, and commercially available antibodies such as Clone: 6H2.B4 (Becton Dickinson) and Clone: 2B5F8 (TECHNE) may be used.

4) After the beads or cup is washed with a buffer solution containing a surfactant such as Tween 20, if possible, measurement is performed by a method suitable for the label, i.e., radioactivity is measured when labeling is attained with a radioactive isotope, voltage is applied and luminescence is measured when labeling is attained with an electrochemiluminescent compound, or enzymatic activity is measured when labeling is attained with an enzyme. Further, when labeling is attained with biotin, labeled avidin is further added, and measurement is performed by a method suitable for the label.

5) A calibration curve is prepared by using values measured for samples containing cytochrome c in known amounts, and amount of cytochrome c contained in the sample is calculated by using the calibration curve.
By the aforementioned steps, amount of cytochrome c in the sample can be quantified.

In the method of the present invention, it is preferred that the cytochrome c antibody reacts with the sample containing cytochrome c in an acidic range (under acidic condition). The term "acidic range" used in the present specification means a pH range in which influence of interference substances in the blood or in serum, which influence the binding of an antibody and cytochrome c, is attenuated. Although the influence of interference substances can be reduced by lowering pH, the binding between the antibody and cytochrome c is also weakened at the same time. Therefore, pH for the immunochemical measurement according to the present invention may be determined to be a pH that satisfies the following conditions.

1. Measurement sensitivity enabling quantification of 10 ng/mL, preferably 1 ng/mL, of cytochrome c in a buffer solution can be afforded.
2. A recovery rate of 70% or higher, preferably 80% or higher, more preferably 90% or higher, can be afforded in a spiked recovery test in the presence of a body fluid.

Since the effect of pH on binding of an antibody and cytochrome c varies depending on the antibody used, pH used in the immunochemical measurement method can be determined to be a pH optimal for each particular antibody. The pH is preferably 7 or lower, more preferably 3 to 6, still more preferably 3.5 to 5, even more preferably 3.5 to 4.5.

Hereafter, a method for determining pH used for the immunochemical measurement of the present invention will be shown specifically. However, the method for determining pH is not limited to this example.

### 1. Influence of pH on measurement sensitivity

Cytochrome c is diluted to 1 to 1000 ng/mL in a buffer solution of pH 3 to 7.5 containing appropriate proteins such as BSA, appropriate salts such as NaCl, appropriate surfactants and so forth as required. When the immunochemical measurement method is a sandwich method, the diluted cytochrome c and an immobilized anti-cytochrome c antibody are reacted, after washing, a labeled anti-cytochrome c antibody is added, and the labeling substance is detected based on an activity corresponding to the labeling substance, such as radioactivity in the case of radioactive labeling and enzymatic activity in the case of enzyme labeling.

When a signal obtained from a specimen containing 10 ng/mL, preferably 1 ng/mL, of cytochrome c is sufficiently stronger than that of a specimen not containing cytochrome c but comprising a buffer solution alone, the used pH can be mentioned as a candidate pH of the buffer solution used for the immunochemical measurement method.

### 2. Influence of pH on added recovery

When cytochrome c in a body fluid corresponding to a specimen used in the immunochemical measurement method, for example, serum, is measured, a known amount of cytochrome c is added to the serum. The amounts of cytochrome c in serum to which cytochrome c is added and serum to which cytochrome c is not added are measured by the immunochemical method, and the ratio of the measured value to the theoretical value is obtained as a recovery rate.

When the addition amount of cytochrome c is represented by A, the measured value of cytochrome c in serum to which cytochrome c is not added is represented by B, and the measured value of cytochrome c in serum to which cytochrome c is added is represented by C, the recovery rate can be calculated in accordance with either of the following equations:
(1) Measured value (C)/Theoretical value (weighted average of A and B)
(2) Increase in measured value by addition of cytochrome c (C - B) /Addition amount (A)

When the recovery rate is 70% is higher, preferably 80% or higher, more preferably 90% or higher, the pH can be mentioned as a candidate pH of a buffer solution used for the immunochemical measurement method.

Further, in the immunochemical measurement of cytochrome c, a method of using a buffer solution in an acidic range is effective not only for the first reaction in which an immobilized antibody and cytochrome c in a specimen are reacted, but also for the second reaction (the step of reacting cytochrome c and a labeled antibody) when the interference substances cannot be completely removed in the first reaction.
The method of the present invention is not limited to a method of using a buffer solution in an acidic for the first reaction.

The buffer solution used for the reaction of cytochrome c in blood and an antibody against cytochrome c in an acidic range is determined taking 1. influence of pH on measurement sensitivity and 2. influence of pH on added recovery mentioned above into account. Type of the buffer solution is not particularly limited so long as the buffer solution can be prepared to be in an acidic condition, and examples thereof include succinate buffer solution, citrate phosphate buffer solution and so forth.

The present invention also relates to use of a kit which comprises reagents for quantifying cytochrome c in blood by using an antibody against cytochrome c (cytochrome c measurement reagents), which is used for testing non-alchoholic steatohepatitis. For example, measurement reagents for measuring cytochrome c by a sandwich method as examples of the cytochrome c measurement reagents include, for example, 1) an anti-cytochrome c antibody-coated cup or an anti-cytochrome c antibody coated beads, and 2) a labeled anti-cytochrome c antibody, and preferably further include 3) a standard cytochrome c solution having a known concentration, 4) a diluent, and 5) a washing solution. Further, in the case of using enzyme labeling, the regents may include 6) a substrate for color development, and 7) a solution for terminating the reaction.

The measurement kit as used in the present invention preferably includes a buffer solution for reacting cytochrome c in blood and an antibody against cytochrome c in an acidic region.

The buffer solution included in the measurement kit may be a buffer solution to be used for the reaction as it is or a buffer solution to be diluted before use. Further, it may be a buffer solution adjusted to have a pH in an acidic region suitable for the present invention by adding an acidic or alkaline solution in an appropriate volume, or a buffer solution attached with an instruction describing such pH adjustment.

The method and kit for measurement of cytochrome c disclosed in the present invention are used for test of non-alcoholic steatohepatitis.

A patient showing a high quantified value of cytochrome c measured by the method and kit for measurement of cytochrome c disclosed in the present invention, usually a patient showing a quantified value exceeding a cutoff value, is identified to be with non-alcoholic steatohepatitis, and the identification can be used as an index for selection of therapies. Although the cutoff value is not particularly limited, so long as non-alcoholic steatohepatitis can be correctly diagnosed, a value deviating by 2SD from average for healthy persons, or 35 ng/mL is usually used.
Cytochrome c level is a favorable index for evaluation of stress-dependent hepatocellular damage, and can serve as a useful clinical test item for quickly and accurately diagnosing non-alcoholic steatohepatitis. Examples

The present invention will be explained more specifically with reference to the following examples. However, the scope of the present invention is not limited by these examples.

### [Reference Example 1] Preparation of anti-cytochrome c monoclonal antibody

Human cytochrome c (TECHNE) in an amount of 110 µg/100 µL and 55 µL of 2 mg/mL ovalbumin dissolved in a 65 mM phosphate buffer solution (pH 7.5) were mixed, to the mixture was added 42 µL of 1 mM glutaraldehyde diluted with a 65 mM phosphate buffer solution (pH 7.5), and the mixture was stirred at room temperature for 2 hours. Then, the mixture was dialyzed against 0.15 M NaCl at 4°C for 48 hours, an adjuvant was prepared with equal amount of FCA, and 0.1 mL thereof was administered to the peritoneal cavities of BALB/C mice to immunize the mice. The immunization was performed in the same manner 3 times every 2 weeks. Two weeks after the third immunization, 50 µg/100 µL of human cytochrome c dissolved in physiological saline was given to the mice by intravenous injection from the caudal vein. Three days later, the spleens were removed from the mice, and spleen lymphocytes were fused with myeloma cell P3X63 Ag8U.1 according to the polyethylene glycol method in a conventional manner. Screening was performed by using human cytochrome c as an antigen to establish a hybridoma producing a monoclonal antibody against human cytochrome c (clone: 27G9).

The established hybridoma was cultured and proliferated in the S-Clone SF-B medium (Sanko Junyaku Co., Ltd.) and inoculated into the peritoneal cavities of BALB/C mice. After 1 week, ascites was collected. IgG was purified from the collected ascites by using protein A to obtain an anti-cytochrome c antibody (27G9 antibody).

### [Reference Example 2] Preparation of anti-cytochrome c antibody-immobilized beads

The anti-cytochrome c monoclonal antibody (clone: 2B5F8 (TECHNE)) was dialyzed against a 0.1 M acetate buffer solution (pH 4.2) containing 0.15 M NaCl, and diluted with a 0.1 M acetate buffer solution (pH 4.2) containing 0.15 M NaCl to have an OD 280 nm of 0.56. The diluted antibody in a volume of 1.67 mL was mixed with 3.36 mL of beads (Dynabeads^{®} M-450 Epoxy, Dynal) washed beforehand three times with 3 mL of a 0.1 M acetate buffer solution (pH 4.2) containing 0.15 M NaCl using a magnet, and the mixture was stirred at room temperature for 17 hours. Then, the beads were suspended in 3 mL of a blocking buffer solution (50 mM Tris-HCl, 1% BSA, 0.15 M NaCl, 0.1% NaN₃, pH 7.5), and the suspension was stirred at room temperature for 7 hours to block the beads. The blocked beads were washed thrice with 3 mL of 150 mM Tris-HCl, 3% BSA, 0.3% treharose, 0.45 M NaCl, 0.03 vol% Tween 20, 0.3% NaN₃, 30 mM EDTA, pH 7.5 and suspended in 12.5 mL of 150 mM Tris-HCl, 0.45 M NaCl, 3% BSA, 0.9% treharose, 0.03 vol% Tween 20, 70 µg/mL mouse IgG, 0.3% NaN₃, pH 7.5. This was diluted three times with purified water before use.

### [Reference Example 3] Preparation of ruthenium complex-labeled anti-cytochrome c antibody

The anti-cytochrome c monoclonal antibody prepared in Reference Example 1 (27G9 antibody) was dialyzed against PBS, and the antibody concentration was adjusted to be in the range of 0.5 to 2 mg/mL. To 1 mL of the antibody was added 12.2 µL of a ruthenium complex (ruthenium(II) tris(bipyridyl)-N-hydroxysuccinimide, IGEN Corp. USA) dissolved in dimethyl sulfoxide at a concentration of 10 mg/mL, and the mixture was stirred at room temperature for 30 minutes. Then, to the mixture was added 50 µL of 2 M glycine, and the mixture was stirred at room temperature for 10 minutes. The mixture was applied to a Sephadex G-25 (Amersham Pharmacia Biotec) column (1.5 cm ϕ x 30 cm) equilibrated beforehand with PBS-3 (10 mM potassium phosphate, 0.15 M NaCl, 0.05% NaN₃, pH 6) and eluted with PBS-3 to collect 1-mL fractions. The OD of each fraction was measured at 280 nm, and fractions of the first peak were collected to obtain an anti-cytochrome c antibody labeled with a ruthenium complex. The antibody concentration was measured by using Micro BCA Protein Assay Kit (PIERCE).

### [Reference Example 4] Preparation of standard human cytochrome c antigen

Standard cytochrome c antigen solutions were prepared by diluting human cytochrome c (TECHNE) with a 0.05 M Tris-HCl buffer solution (pH 7.8) containing 5% BSA, 0.15 M NaCl and 0.1% NaN₃ to 3000 ng/mL, 1000 ng/mL, 100 ng/mL, 10 ng/mL and 5 ng/mL.

### [Example 1] Measurement of cytochrome c

A solution for diluting samples (0.1 M succinate buffer solution containing 0.15 M NaCl, 15 mM EDTA, 2 volume % Lipidure (registered trademark) BL802 (Nippon Oil & Fats Co., Ltd.), 2 volume % Lipidure (registered trademark) BL405 (Nippon Oil & Fats Co., Ltd.) and 0.1% NaN₃, pH 4.0) in a volume of 200 µL was put into a reaction tube for Picolumi^{R} 8220 (Sanko Junyaku Co., Ltd.), and 20 µL of a sample was injected into the tube.

The following measurement was performed by using an electrochemiluminescent enzyme immunoassay apparatus, Picolumi^{R} 8220 (Sanko Junyaku Co., Ltd.).

The anti-cytochrome c antibody-immobilized beads prepared in Reference Example 2 in a volume of 25 µL were added to the reaction tube, allowed to react for 9 minutes, and washed twice with 350 µL of Picolumi^{R} BF washing solution (Sanko Junyaku Co., Ltd.). Then, 200 µL of the ruthenium complex-labeled anti-cytochrome c antibody diluted with the diluent for ruthenium complex-labeled anti-cytochrome c antibody (0.05 M Tris-HCl, 1% BSA, 0.15 M NaCl, 0.3% of trehalose, 0.01 volume % Tween 20, 0.3% NaN₃, pH 7.5) prepared in Reference Example 3 to 0.5 to 2 µg/mL were added to the beads, and the reaction was allowed for 9 minutes. The beads were washed twice with 350 µL of Picolumi^{R} BF washing solution, then 300 µL of Picolumi^{R} luminescence electrolytic solution (Sanko Junyaku Co., Ltd.) was added to the beads, and luminescence count value was measured.

The results were plotted with human cytochrome c standard antigen concentrations as abscissa against the luminescence count values of human cytochrome c standard antigen as ordinate to prepare a standard curve, and the amounts of cytochrome c contained in the samples were calculated from the luminescence count values on the basis of the standard curve.

### [Example 2] Assay of cytochrome c in serum of non-alcoholic steatohepatitis patients

Cytochrome c amounts in serum of healthy persons and non-alcoholic steatohepatitis patients were quantified by using an established ELISA system for cytochrome c.

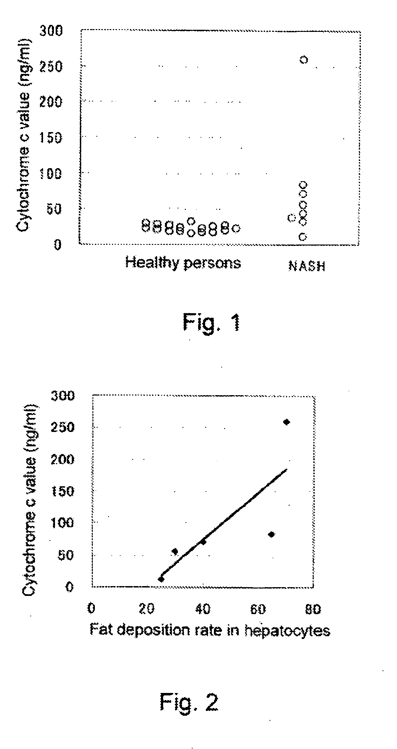

As a result, as shown in Fig. 1, non-alcoholic steatohepatitis patients showed high quantified cytochrome c values of 30 ng/mL or higher, whereas healthy persons showed the values of 20 to 30 ng/mL. Thus, it was possible to determine whether a patient suffered from non-alcoholic steatohepatitis or not by quantifying cytochrome c in blood.

It was demonstrated that test of non-alcoholic steatohepatitis based on quantified values of serum cytochrome c was possible.

### [Example 3] Correlation of liver tissue fat rates and quantified values of serum cytochrome c in non-alcoholic steatohepatitis patients

Fat deposition rates in hepatocytes collected from non-alcoholic steatohepatitis patients by biopsy and quantified values of serum cytochrome c in the patients were compared.

The fat deposition rates in the hepatocytes of the patients were calculated from areas of fat deposition in liver tissues collected by biopsy and stained with HE staining (hematoxylin and eosin staining) and AZAN staining. Further, serum cytochrome c levels in the patients were quantified by the method described above. As a result, as shown Fig. 2, the fat deposition rates in the hepatocytes and the quantified values of serum cytochrome c showed positive correlation (R² = 0.6452), and it was demonstrated that quantified value of cytochrome c serves as a favorable index of fat deposition in hepatocytes and stress generated thereby.

It was demonstrated that, by measuring blood cytochrome c, fat deposition in hepatocytes and stress accompanying it can be non-invasively evaluated without biopsy of the liver, and thus blood cytochrome c could serve as a favorable index of non-alcoholic steatohepatitis.

### Industrial Applicability

According to the present invention, it became possible to non-invasively diagnose non-alcoholic steatohepatitis by quantifying blood cytochrome c.

## Claims

1. An in vitro method of testing for the presence of non-alcoholic steatohepatitis, which comprises the step of quantifying cytochrome c in collected blood.

2. The test method for non-alcoholic steatohepatitis according to claim 1, which comprises:
(1) the step of quantifying cytochrome c in blood, and
(2) the step of identifying non-alcoholic steatohepatitis when the quantified value of cytochrome c is high.

3. The method according to claim 1 or 2, wherein the step of quantifying cytochrome c in blood is a step of quantifying cytochrome c using an antibody against cytochrome c.

4. The method according to claim 3, wherein the step of quantifying cytochrome c using an antibody against cytochrome c is a step comprising reacting cytochrome c in blood and the antibody against cytochrome c under acidic conditions.

5. A use of a kit in the method according to any one of the claims 1-4, wherein said kit comprises a reagent for quantifying cytochrome c in blood using an antibody against cytochrome c.

6. The use according to claim 5, wherein said kit comprises a buffer solution for reacting cytochrome c in blood and the antibody against cytochrome c under acidic conditions.

## Patentansprüche

1. Ein In-vitro-Verfahren zum Prüfen des Vorliegens von nichtalkoholischer Steatohepatitis, das den Schritt des Quantifizierens von Cytochrom c in gesammeltem Blut umfasst.

2. Das Prüfverfahren für nichtalkoholische Steatohepatitis nach Anspruch 1, das umfasst:
(1) den Schritt des Quantifizierens von Cytochrom c in Blut und
(2) den Schritt des Identifizierens nichtalkoholischer Steatohepatitis, wenn der quantifizierte Wert von Cytochrom c hoch ist.

3. Das Verfahren nach Anspruch 1 oder 2, wobei der Schritt des Quantifizierens von Cytochrom c in Blut ein Schritt des Quantifizierens von Cytochrom c ist, bei dem ein Antikörper gegen Cytochrom c verwendet wird.

4. Das Verfahren nach Anspruch 3, wobei der Schritt des Quatifizierens von Cytochrom c, bei dem ein Antikörper gegen Cytochrom c verwendet wird, ein Schritt ist, der das Reagieren von Cytochrom c in Blut und des Antikörpers gegen Cytochrom c unter sauren Bedingungen umfasst.

5. Eine Verwendung eines Kits bei dem Verfahren nach einem der Ansprüche 1 bis 4, wobei der Kit ein Reagenz für die Quantifizierung von Cytochrom c in Blut unter Verwendung eines Antikörpers gegen Cytochrom c aufweist.

6. Die Verwendung nach Anspruch 5, wobei der Kit eine Pufferlösung zum Reagieren von Cytochrom c in Blut und des Antikörpers gegen Cytochrom c unter sauren Bedingungen aufweist.

## Revendications

1. Procédé in-vitro pour détecter la présence de stéato-hépatite non-alcoolique comprenant une étape consistant à quantifier le cytochrome c dans du sang recueilli.

2. Procédé pour détecter la stéato-hépatite non alcoolique conforme à la revendication 1, comprenant :
(1) l'étape consistant à déterminer la quantité de cytochrome c dans le sang, et
(2) l'étape consistant à identifier la stéato-hépatite non-alcoolique lorsque la quantité de cytochrome c décelée, est élevée.

3. Procédé conforme à la revendication 1 ou 2,
selon lequel
l'étape consistant à déterminer la quantité de cytochrome c dans le sang, est une étape consistant à déterminer la quantité de cytochrome c en utilisant un anticorps anticytochrome c.

4. Procédé conforme à la revendication 3,
selon lequel
l'étape consistant à déterminer la quantité de cytochrome c en utilisant un anticorps anticytochrome c, est une étape comprenant la mise en réaction du cytochrome c du sang et de l'anticorps anticytochrome c dans des conditions acides.

5. Utilisation d'un kit pour la mise en oeuvre du procédé conforme à l'une quelconque des revendications 1 à 4,
selon laquelle
ce kit renferme un réactif pour déterminer la quantité de cytochrome c dans le sang en utilisant un anticorps anticytochrome c.

6. Utilisation conforme à la revendication 5,
selon laquelle
le kit renferme une solution tampon pour faire réagir le cytochrome c du sang et l'anticorps anticytochrome c dans des conditions acides.
